# EUROPEAN PATENT SPECIFICATION

(11) **EP 4 355 195 B1**
(45) Date of publication and mention of the grant of the patent: **01.04.2026**
(21) Application number: 22728124.3
(22) Date of filing: 05.05.2022
(51) Int. Cl.: A61B 5/0285, A61B 5/0265, A61B 5/107, A61B 5/026, A61B 5/0507, A61B 5/00

(54) **PULSE WAVE VELOCITY**
PULSWELLENGESCHWINDIGKEIT
VITESSE DE L'ONDE PULSÉE

(30) Priority: 18.06.2021 WO PCT/SE2021/050608
(43) Date of publication of application: 24.04.2024
(73) Proprietor: Neko Health AB, 182 33 Danderyd (SE)
(72) Inventor: WINDÅ, Mattias, 185 39 Vaxholm (SE); HAINZL, Richard, 191 41 Sollentuna (SE)
(74) Representative: Kransell & Wennborg KB
(86) International application number: PCT/EP2022/062201
(87) International publication number: WO 2022/263053

(56) References cited:
- CN-A- 108 852 307
- LI YANLU ET AL: "Silicon photonics-based laser Doppler vibrometer array for carotid-femoral pulse wave velocity (PWV) measurement", BIOMEDICAL OPTICS EXPRESS, vol. 11, no. 7, 22 June 2020 (2020-06-22), United States, pages 3913, XP055963855, ISSN: 2156-7085, Retrieved from the Internet <URL:https://opg.optica.org/DirectPDFAccess/0E0F1D68-3064-4F66-B5AFA9D8A2319C75_432824/boe-11-7-3913.pdf?da=1&id=432824&seq=0&mobile=no> [retrieved on 20220922], DOI: 10.1364/BOE.394921
- DE MELIS M. ET AL: "A Noncontact Approach for the Evaluation of Large Artery Stiffness: A Preliminary Study", AMERICAN JOURNAL OF HYPERTENSION, vol. 21, no. 12, 1 December 2008 (2008-12-01), United States, pages 1280 - 1283, XP055890804, ISSN: 0895-7061, Retrieved from the Internet <URL:https://academic.oup.com/ajh/article-pdf/21/12/1280/396794/21_12_1280.pdf> [retrieved on 20220922], DOI: 10.1038/ajh.2008.280
- LAUTESLAGER TIMO ET AL: "Coherent UWB Radar-on-Chip for In-Body Measurement of Cardiovascular Dynamics", IEEE TRANSACTIONS ON BIOMEDICAL CIRCUITS AND SYSTEMS, IEEE, US, vol. 13, no. 5, 1 October 2019 (2019-10-01), pages 814 - 824, XP011754475, ISSN: 1932-4545, [retrieved on 20191105], DOI: 10.1109/TBCAS.2019.2922775

## Description

### TECHNICAL FIELD

The present disclosure relates to a method of calculating a Pulse Wave Velocity (PWV) in a live body.

### BACKGROUND

A human body can be non-invasively examined using for example: penetrating photons (x-ray/CT, radio waves), electrical and magnetic fields (MRI), nuclear emissions (PET, Gamma camera), emitted and reflected photons (IR and visible light), ultra-sound (imaging/doppler) and electrical potential (EEG, EKG).

Such techniques are well known and in use. However, several of these techniques for observation of body internals (CT, MRI, PET) require large machines that can only be installed into hospitals or larger clinics. Also, devices using or triggering ionizing radiation like X-rays and PET, cannot be used on a large part of the population due to the side-effects of such radiation.

Speed is also of importance for a multimodal modelling system, since the test subject has limited ability to remain still for a longer period of time.

The Pulse wave velocity (PWV) is the velocity at which the blood pressure pulse propagates through the circulatory system of a human or animal live body, usually an artery or a combined length of arteries. PWV is used clinically as a measure of arterial stiffness and can be measured non-invasively, e.g. with measurement of carotid to femoral PWV (cfPWV).

Li Yanlu et al. "Silicon photonics-based laser Doppler vibrometer array for carotid-femoral pulse wave velocity (PWV) measurement", Biomedical Optics Express, vol. 11, no. 7, 22 June 2020, page 3913, discloses using handheld PWV sensors with miniaturized laser Doppler vibrometer (LDV) arrays for assessing aortic stiffness.

De Melis M. et al. "A Noncontact Approach for the Evaluation of Large Artery Stiffness: A Preliminary Study", American Journal of Hypertension, vol. 21, no. 12, 1 December 2008, pages 1280-1283, relates to estimating carotid-femoral PWV for evaluating arterial stiffness.

Lauteslager Timo et al. "Coherent UWB Radar-on-Chip for In-Body Measurement of Cardiovascular Dynamics", IEEE Transactions on Biomedical Circuits and Systems, IEEE, US, vol. 13, no. 5, 1 October 2019, pages 814-824, relates to measuring cardiovascular dynamics using UWB radar-on-chip.

CN 108 852 307 discloses a non-contact non-invasive arteriosclerosis detecting device.

### SUMMARY

It is an objective of the present invention to provide an efficient and automated way of measuring cfPWV in a live body, e.g. of a human or an animal subject.

In an aspect of the present invention, there is provided a method of calculating a PWV in a live body. The method comprises obtaining a geometrical model of the body based on acquired sensor data. The method also comprises, based on the obtained model, automatically positioning a first vibration sensor in relation to a neck area of the body. The method also comprises, based on the obtained model, automatically positioning a second vibration sensor in relation to a pelvis area of the body. The method also comprises detecting a carotid artery pulse by means of the first vibration sensor and a femoral artery pulse by means of the second vibration sensor, both the detected carotid artery pulse and the detected femoral artery pulse resulting from the same blood pressure pulse wave. The method also comprises calculating the cfPWV based on a time difference between the detected carotid pulse and the detected femoral pulse, and on a spatial distance between the neck area and the pelvis area.

In another aspect of the present invention, there is provided a system comprising a sensor for acquiring data of a live body, a first vibration sensor, a second vibration sensor, processing circuitry, and storage storing instructions executable by said processing circuitry whereby said system is operative to perform an embodiment of the method of the present disclosure.

In another aspect of the present invention, there is provided a system comprising means for obtaining a geometrical model of a live body, means for, based on the obtained model, automatically positioning a first vibration sensor in relation to a neck area of the body, means for, based on the obtained model, automatically positioning a second vibration sensor in relation to a pelvis area of the body, means for detecting a carotid artery pulse by means of the first vibration sensor and a femoral artery pulse by means of the second vibration sensor, both the detected carotid artery pulse and the detected femoral artery pulse resulting from the same blood pressure pulse wave, and means for calculating the cfPWV based on a time difference between the detected carotid pulse and the detected femoral pulse, and on a spatial distance between the neck area and the pelvis area.

By means of the model of the body, typically a digital model stored electronically as data in a data storage, the vibration sensors can be positioned automatically to detect pulses in the respective arteries. The pulse is caused by a pulse wave which is a pressure wave generated by the heart (systole) which move the arterial walls. These movements of the arterial walls are detected by the vibration sensors. For cfPWV measurements, a standard way of estimating the distance travelled by the pulse wave is 80% of the direct spatial distance between the respective points of the common carotid artery in the neck and the femoral artery in the groin where the pulse is detected. The spatial distance, preferably the spatial direct distance between where the carotid pulse is detected and where the femoral pulse is detected on the body, may preferably be determined or estimated from the obtained model of the body, but may alternatively or additionally be measured manually.

It is to be noted that any feature of any of the aspects may be applied to any other aspect, wherever appropriate. Likewise, any advantage of any of the aspects may apply to any of the other aspects. Other objectives, features and advantages of the enclosed embodiments will be apparent from the following detailed disclosure, from the attached dependent claims as well as from the drawings.

Generally, all terms used in the claims are to be interpreted according to their ordinary meaning in the technical field, unless explicitly defined otherwise herein. All references to "a/an/the element, apparatus, component, means, step, etc." are to be interpreted openly as referring to at least one instance of the element, apparatus, component, means, step, etc., unless explicitly stated otherwise. The steps of any method disclosed herein do not have to be performed in the exact order disclosed, unless explicitly stated. The use of "first", "second" etc. for different features/components of the present disclosure are only intended to distinguish the features/components from other similar features/components and not to impart any order or hierarchy to the features/components.

### BRIEF DESCRIPTION OF THE DRAWINGS

Embodiments will be described, by way of example, with reference to the accompanying drawings, in which:
Fig 1 illustrates how visible and thermal cameras may be used in a modelling system 100 for a standing test subject, in accordance with some embodiments of the present invention.
Fig 2 illustrates how visible and thermal cameras may be used in a modelling system 100 for a lying test subject, in accordance with some embodiments of the present invention.
Fig 3 illustrates an embodiment of a module 400 for model acquisition comprising a structured light scanner and a colour camera, in accordance with some embodiments of the present invention.
Fig 4 illustrates an embodiment of structured light scanner combined with high intensity light fixtures, in accordance with some embodiments of the present invention.
Fig 5 illustrates an example of an array of laser vibrometers, in accordance with some embodiments of the present invention.
Fig 6 Illustrates an arrangement with laser and microwave vibrometers for measuring a PWV of a test subject, in accordance with some embodiments of the present invention.
Fig 7 illustrates several locations on the body of a test subject which may be of interest for measuring a PWV, in accordance with some embodiments of the present invention.
Fig 8 illustrates an arrangement for PWV measurements, comprising an array of vibrometers, e.g. laser vibrometers, over the neck area and an array of vibrometers, e.g. microwave vibrometers, over the pelvis area when the test subject is lying down, in accordance with some embodiments of the present invention.
Fig 9 illustrates another arrangement for PWV measurements, comprising an array of vibrometers, e.g. laser vibrometers, over the neck area and an array of vibrometers, e.g. microwave vibrometers, over the pelvis area when the test subject is lying down, in accordance with some embodiments of the present invention.
Fig 10 is a flow chart illustrating some embodiments of the method of the present disclosure.

### DETAILED DESCRIPTION

Embodiments will now be described more fully hereinafter with reference to the accompanying drawings, in which certain embodiments are shown. However, other embodiments in many different forms are possible within the scope of the present disclosure. Rather, the following embodiments are provided by way of example so that this disclosure will be thorough and complete, and will fully convey the scope of the disclosure to those skilled in the art. Like numbers refer to like elements throughout the description.

The model of the body may be a 2D model or a 3D model, of the whole body or of a part of the body, as long as it is adequate for basing the automatic positioning of the vibration sensors for detecting the respective carotid and femoral artery pulses. Typically, the model is of an outside surface (typically provided by skin, but possibly by underwear) of the body. The sensor data may be acquired by any suitable means (such as comprising the sensor discussed herein), e.g. a conventional means such as by optical or infrared camera(s), e.g. 3D cameras, time-of-flight cameras and/or colour, e.g. high-resolution, cameras, microwaves or lasers (e.g. LIDAR), or any combination thereof, to provide the model of the body. In some embodiments, the model is in the form of, or comprises, a point cloud. In some embodiments, infrared camera(s) are used to estimate the position of a carotid or femoral artery close to the skin where a pulse may easily be detected by the vibration sensor.

The acquiring of sensor data of the body to obtain the model may e.g. comprise any of gathering spectral information, texture, and topography by combining 2D imaging at multiple focus depths, with a 3D point cloud. A 3D point cloud enables absolute measurement of body features and for aligning the other imaging systems. The point cloud is a set of data points in space representing the 3D shape of the body, where each point represents a position on the surface of the body and is defined by coordinates. The 3D point cloud provides an absolute coordinate system through which other measurements and images can be aligned. This enables pixels and/or voxels to be compared within the same physical surface and/or volume representing the body.

The 3D point cloud may be created from a separate system such as a structured light based 3D imaging system, or via triangulation of multiple overlapping images from 2D image cameras. In yet another embodiment the point cloud can be acquired using lasers/microwave radars, e.g. mounted on moveable rings pointing inwards toward the body. When such rings move to cover the whole body, the distances captured by the lasers/microwave radars can be used to create the point cloud.

In an embodiment such a point cloud can consist of 3D triangles (tetrahedral mesh) to cover the skin surface. In another embodiment the point cloud consists of voxels forming the surface of the test subject.

The point cloud can be used to geometrically overlay other measured values (e.g. high-resolution images, thermal images, Ultra-Wide Bandwidth (UWB) measurements, vibrational measurements) onto a 3D model of the subject.

Additionally or alternatively, thermal imaging may be used as, or as part of, the acquiring of sensor data of the body. Thermal imaging may imply imaging of heat radiation from the body, based on IR (infrared) cameras. Measuring distribution of heat generation to detect blood vessels. These thermal images can then be overlayed on top of and in combination with the other measurements.

By using vibration sensor(s), e.g. laser vibrometer(s) and/or microwave radar(s)/vibrometer(s), it may be possible to measure pulse wave shapes at different body locations. This can be used to calculate the pulse wave velocity (PWV) and perform other pulse wave analyses. Using a vibration sensor (herein also called vibrometer), it may also be possible to record heart sounds. Vibrometers can also be used to perform myography and respiratory analysis.

For instance, more than one vibrometer, e.g. laser of a vibrometer, each aimed at respective different parts of the body, can be used for measuring the pulse wave velocity originating from the heart beats. The pulse wave velocity is an indication of the arterial stiffness. Higher velocity implies higher arterial stiffness.

By means of the model, e.g. comprising or consisting of a point cloud representing the skin surface of the body, any acquired colour images, thermal images, model of internal organs, and/or vibrational measurements can be accurately related in space (i.e. spatially) to the body allowing the body to be modelled with the acquired information. A point cloud can be used, or based upon, by correlating information (images, measurements etc.) to a common coordinate system of the body provided by the point cloud. Such correlated information can then be a source for scheduling camera focus and/or point of view, locating neck and pelvis for vibrational measurements, calculating a skin height map for UWB measurements etc.

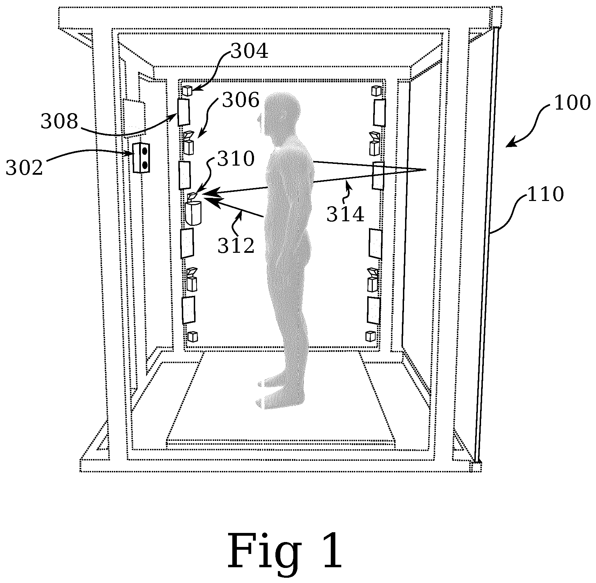

Figure 1 illustrates how visible and thermal photons can be collected by a modelling system 100 for a standing test subject. To simplify the figure, only the cameras and light emitters mounted on the remote pillars are drawn. In reality any of the pillars or other structural elements may carry the cameras and/or light emitters.

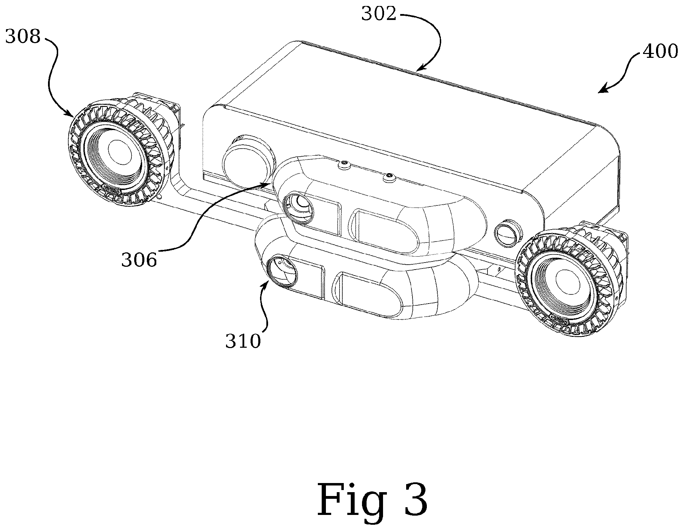

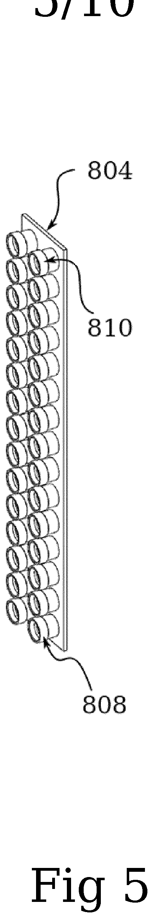

The model, e.g. a point cloud, may be be acquired using a structured light 3D scanner 302. Such a device 302 comprises a structured light projector and a camera. The camera records the different light patterns projected on the body by the projector and can construct a 3D model, e.g. point cloud, based on this information. An example of such a commercially available camera is the Ajile DepthScan^{™} 3D imaging system. The structured light 3D scanner is further illustrated in figures 3 and 5.

Such a model is limited to the point of view of the device 302. In figure 1, a single device 302 is shown. However a plurality of such devices 302 may be positioned, e.g. on all the pillars, around the body to capture the entire body.

It is may additionally or alternatively be possible to use a passive model/point cloud acquisition system using multiple wide field of view cameras 304. Using two or more of these cameras 304, it is possible to construct a model/point cloud using photogrammetry analysis.

In another embodiment it is possible to combine 3D model data acquired from the structured light scanner 302 with data from the wide view cameras 304, to create a combined 3D model. In the figure the structured light scanner 302 can observe the coronal plane viewing the chest and head areas. The model of the remainder of the body may be covered by the photogrammetry analysis using the wide view cameras 304.

In another embodiment it is possible to use a camera with time of flight measurements to acquire the model. Examples of such a camera is the Azure Kinect DK^{™} depth camera which continuously transmits an amplitude modulated continuous wave in the near infra-red spectrum onto the body and then records the time it takes for the light to travel from the camera to the body and back.

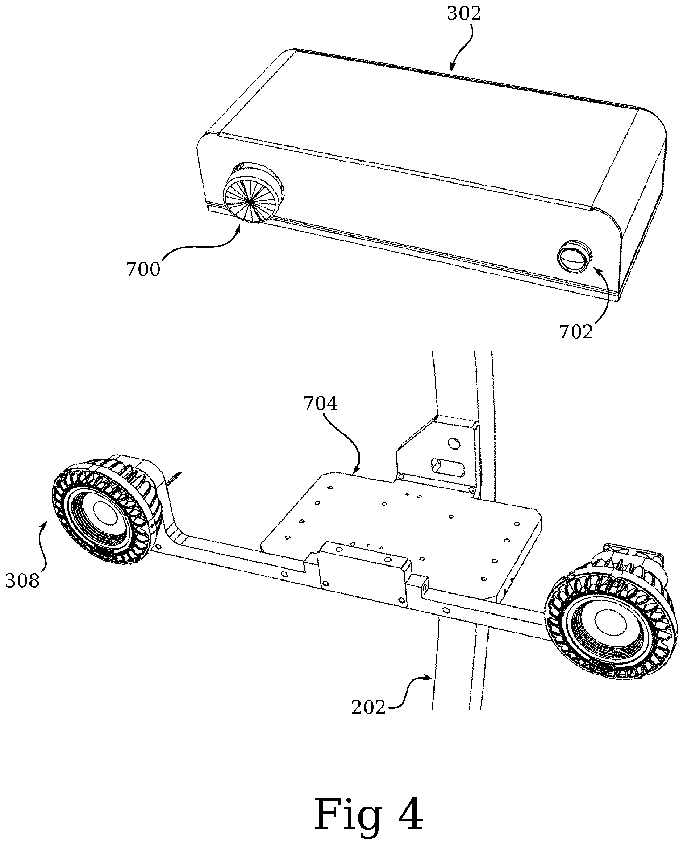

The colour camera 306 may have a narrow field of view and a shallow depth of field designed to take multiple overlapping skin images with a high-resolution. A mirror may be used to select the desired field of view. A focusing device may then be used to take a stack of images using different focal distances for the selected field of view. The stack is then merged into a single image using a focal plane merging algorithm. The camera 306 for the visible light spectrum is further illustrated in figure 4a.

The colour camera may need a significant amount of lighting to acquire high quality images and the LED screen 308 is one of 8 light emitters drawn in the figure with 8 more not drawn, presented as an example of lighting.

Thermal camera 310 is an example of a thermal camera. Such a camera may have an appearance similar to the camera 306. But high quality thermal cameras are typically larger and significantly more expensive than a colour camera. The thermal camera 310 is further illustrated in figure 4b.

A thermal camera 310 may view the front of the body of a subject through direct thermal emissions 312, but may additionally or alternatively view the back of the body through a reflection 314 in a glass panel/mirror 110. For a standing test subject it may be preferable to be able to image both back and front simultaneously. A fast acquisition may be important since the test subject may move involuntarily when standing. Uncorrected movements can worsen the positional errors and the continuity of testing.

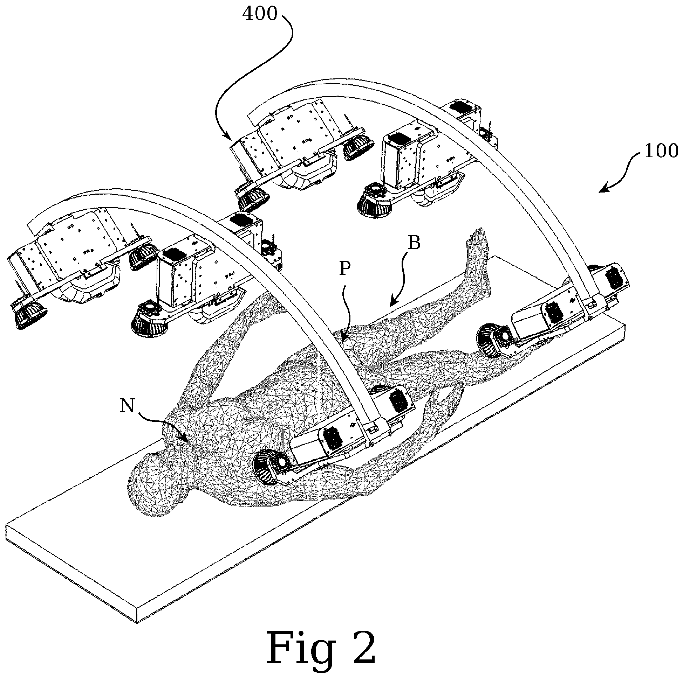

Figure 2 illustrates how a model, e.g. comprising a point cloud, visible and thermal photons may be collected by the modelling system 100 for a lying subject. A module 400 may contain a light emitter 308, a colour camera 306, a thermal camera 310, and a structured light scanner 302. A plurality of modules 400 may be used. In the example of figure 2, six modules 400 are positioned so as to cover the body.

Figure 3 illustrates an embodiment of a module 400 for model acquisition. In the example of figure 3, the structured light scanner 302 is positioned behind the colour camera 306 to minimize the parallax errors between the structured light scanner 302 and the colour camera 306. The light emitters 308 may be high intensity LED light emitters. The thermal camera 310 may be positioned next to the colour camera 306.

Figure 4 illustrates a model/point cloud acquisition device and lighting fixtures. The structured light scanner 302 comprises a camera 700 and structured light projector 702. The structured light scanner 302 is mounted on the fixture 704. The high intensity light emitters 308 are mounted on the same fixture. The fixture may be mounted on a structural support beam 202.

Figure 5 illustrates an example of an array of laser vibrometers, which may be comprised in the first and/or second vibration sensor. A circuit board has laser vibrometers 808, 810. The laser vibrometers can measure vibrations in the skin surface of the body. Such vibrations can indicate heart beats and valve function, as well as artery and vein pulses. Each laser vibrometer may also be used to measure the distance to the skin surface.

In figure 5, the array 804 of laser vibrometers of a vibration sensor form an array of measurements points on the test subject's body. With such an array positioned around the neck, then the speed of the pulse wave (moving through a carotid artery or a jugular vein in the neck) can be directly measured by observing how the vibrations propagates from one vibrometer to the next. In the figure, the pulse may be detected as moving from vibrometer 808 through all intermediate vibrometers and finally detected in vibrometer 810.

More pliable vessels will give a slower pulse wave velocity and stiffer vessels will give a higher pulse wave velocity.

In another embodiment, a single pulse peak is found using the vibrometer array or a single vibrometer 808 or 810. This pulse peak is correlated with the pulse peak in the femoral arteries measured by a vibration sensor, comprising a vibrometer or vibrometer array, positioned around the pelvis of the body. Since the location of femoral measurement is further away from the heart than the location of the carotid measurement, the delay after the carotid pulse has been detected until the femoral pulse has been detected can be used to calculate the carotid-femoral pulse wave velocity (cfPWV).

A single laser vibrometer 808 or 810 is sensitive but has a small pickup size which means that it must be correctly positioned over the artery. In an embodiment such a pickup spot can be positioned using a mirror controlled by a coil. When a "coil" is referred to herein, intended is a coil used to actuate the movement of the mirror by means of electromagnetic force, e.g. similar to a voice coil in a speaker. In such an embodiment the positioning of the pickup spot can be done using the model/point cloud. Then, the mirror can position the laser pick-up spot on the skin of a test subject based on the model, e.g. based on thermal, colour and/or 3D images in the model.

Regardless of whether a single laser vibrometer or an array of laser vibrometers is used, automatic positioning of the first or second vibration sensor comprising said laser vibrometer(s) may include positioning the respective pick-up spot(s) of the laser vibrometer(s) by means of a movable mirror, e.g. actuated by a coil. Positioning of the pick-up spot(s) by means of a mirror may for instance be a way of fine tuning the positioning of the vibration sensor.

In an embodiment using a 1D array of vibrometers or a 2D array 804 of vibrometers 808 and 810, then the model/point cloud can be used to automatically position the array over the neck of the body.

Arteries being reasonably close to the skin also shows an increase in thermal emissions, in particular so for the carotid and femoral arteries. In another embodiment the thermal image acquired using the thermal cameras 310 may also be used as input for guidance of the vibration sensor, e.g. laser vibrometer or microwave radar vibrometer, pickup area for the arteries.

In other embodiments it is possible to use a microwave radar, e.g. of 24 GHz, which will give a larger pickup area size but is less sensitive than laser vibrometers. In yet another embodiment a microwave radar of 120 GHz can be used which positions itself between the 24 GHz radar and the laser vibrometer in regards to sensitivity and pickup size. In some embodiments, to improve sensitivity, it may be convenient to use a relatively high frequency for the microwave radar vibrometer(s) within the range of 80-200 GHz, preferably within the range of 120-150 GHz, more preferably within the range of 136-148 GHz. The specificity of a microwave radar vibrometer may be improved by narrowing its microwave been emitted towards the area of the body (B), thus also reducing its pick-up area on the skin or other surface (e.g. if clothed) of the body. To narrow the microwave beam, a microwave lens may be arranged in the microwave radar vibrometer, through which the microwave beam travels after being generated. If an array, e.g. a 1D or 2D array, of a plurality of microwave radar vibrometer is used in at least one of the vibration sensors, preferably the second vibration sensor, it may be advantageous to position the vibration sensor at a suitable distance from the area, e.g. the pelvis area, at which the pulse should be detected, such that the respective pick-up areas of all the microwave radar vibrometers in the array overlap to cover the whole area (e.g. surface) of the body to be covered. However, the overlap is preferably not larger than needed to cover the whole area, to reduce the pick-up area and thus improve the specificity of the vibrometers. The overlap can be adjusted by positioning the vibration sensor a suitable, typically predetermined, distance from the area/surface of the body. In some embodiments, the positioning of the first vibration sensor and/or the second vibration sensor comprises positioning the vibration sensor at a distance from the area of the body in relation to which the vibration sensor is positioned which is within the range of 10-100 cm, e.g. 30-50 cm. It is also possible to combine a microwave radar with a laser vibrometer in a vibration sensor.

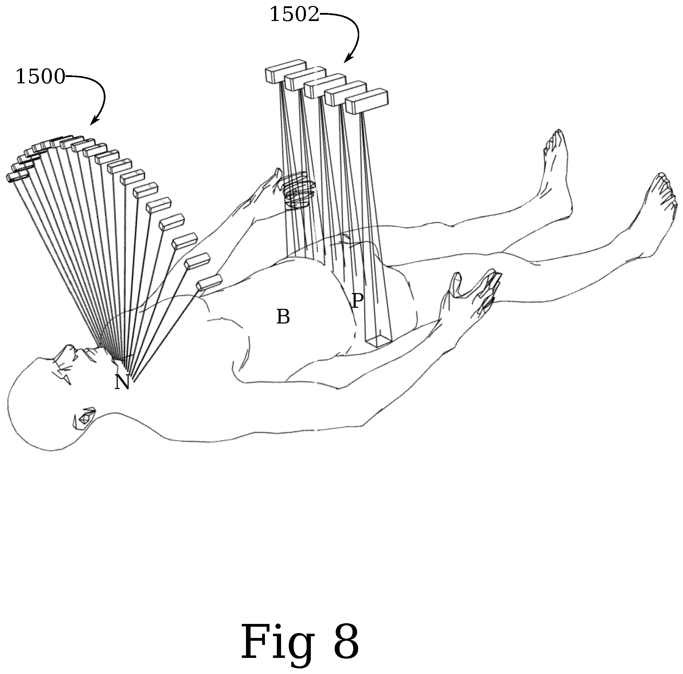

Each of the vibration sensors may thus comprise any combination of laser vibrometers and microwave radar vibrometers. In an embodiment of figure 8, an array 1500 of laser vibrometers are pointing towards the neck area and the array 1502 of microwave radars are pointing towards the pelvis area. This setup has an advantage since the microwave radar is not as affected by clothing as laser vibrometers and therefore the test subject can keep their underwear on.

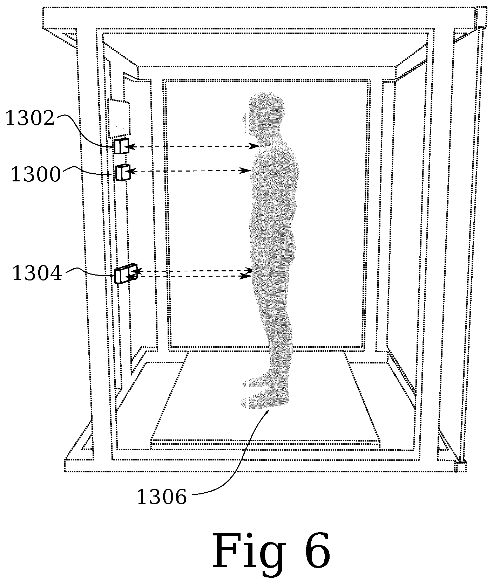

Figure 6 illustrates an embodiment with laser vibrometers and microwave radars capable of measuring the test subject's Pulse Wave Velocity (PWV). In an embodiment a laser vibrometer 1300 transmits a laser beam towards the test subject's thorax where the heart vibrations emanate the most.

The reflected laser light is measured and from the minute movements of the skin, caused by the vibrations of the heart, the pulse can be measured. Such a vibrometer can also be used to record heart sounds and heart movements. In another embodiment, a microwave radar is used for the thorax measurements instead of the laser vibrometer.

A second laser vibrometer 1302 may measure the pulse in in one or both of the common carotid arteries and one or two laser vibrometers (or microwave radars) 1304 may measure one or both of the respective pulses in the femoral arteries.

In an embodiment, the pulse signal can be measured at the thorax (heart, e.g. at apex) and the time between the thorax pulse and the femoral (groin at pelvis) pulses can be used to calculate how fast the pulse wave has propagated through the vessels.

However, the thorax measurement 1300 typically has less precise pulse wave measurements compared to the carotid and femoral measurements, due to the rib cage and the distance between the heart and the skin surface. Therefore, as an alternative, one can measure the timing difference between the pulse detected in the carotid 1302 and femoral arteries 1304 (cfPWV). It may additionally or alternatively be possible to measure the pulse in either or both of the feet using a vibrometer 1306 in the platform.

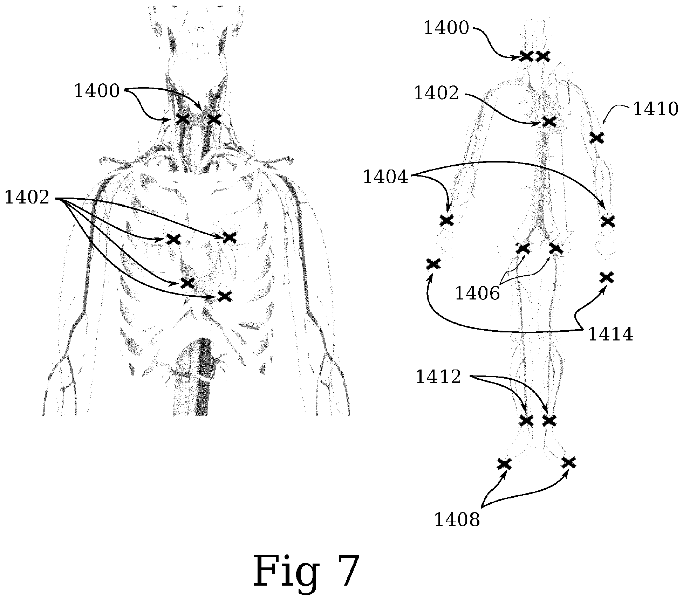

Figure 7 illustrates several locations of interest for measuring the PWV. The carotid arteries 1400 have a good signal to noise ratio for pulse measurements. By measuring at different thorax locations 1402 it is possible to estimate the heart movement and position. The femoral arteries 1406 also have good signal to noise ratio since they, like the carotid arteries generate vibrations near the skin surface. Other arteries like the radial arteries 1404, the brachial arteries 1410 and the fibular pereonal/posterior tibial arteries 1412, are less suited for remote sensing using a laser or radar vibrometer. The brachial artery and the radial artery are however the more common arteries used for blood pressure measurements using pressure cuff.

The pulse can alternatively be measured at the fingertips 1414 and the feet 1408. The pulse shape at the fingertips/feet is different from the pulse shape at a carotid or thorax, why the task of finding a suitable reference point (peak/foot/gradient) requires more analysis in a post processing step.

Figure 8 illustrates an embodiment for PWV measurements where the test subject is lying down. A 1D array of laser vibrometers 1500 is positioned over the neck area N and a 1D array of microwave radar vibrometers 1502 is positioned over the pelvis area P of the body B of the subject.

The 1D array 1500 reduces the need for a precise position of a single laser vibrometer. Instead the pulse signal can be extracted from the array of measurements. If the array is 2D 804 as shown in figure 5, then the pulse can even be monitored while it is traversing the carotid artery.

Such a 1D array 1500 can additionally or alternatively be used to extract the jugular vein pulse. Normally the jugular vein pulse is difficult to extract since the carotid artery in the neck obscures the vein pulse measurement. However, with a 1D array it may be possible to calculate the difference between the pulse measurements on the right-hand side of the neck with the measurements on the left hand side. The human body normally has a weaker jugular vein pulse on the left side of the neck, but the carotid artery pulse is more or less the same on both sides. This difference can be used in an embodiment to extract the jugular vein pulse using a 1D (or 2D) array of sensors.

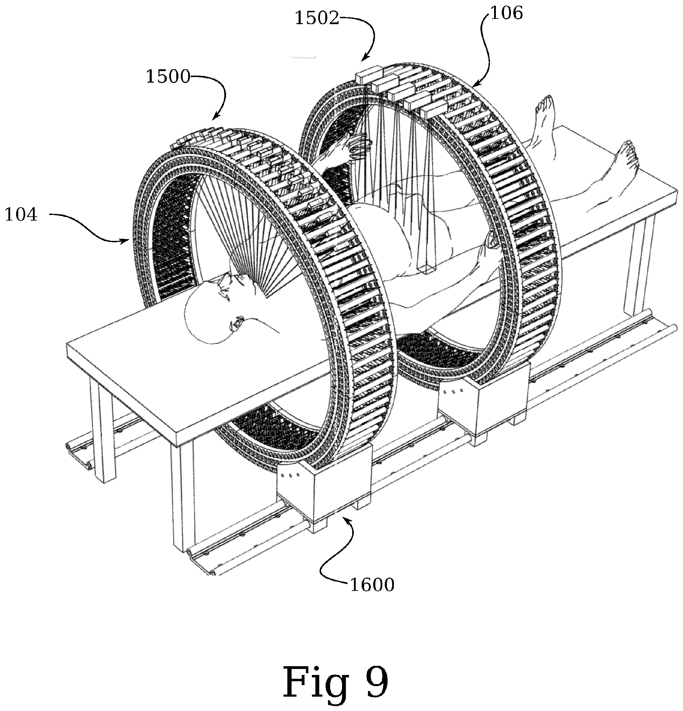

Figure 9 illustrates how the lasers 1500 and radars 1502 in another embodiment can be mounted on rings 104, 106. The rings may initially be positioned above the head and below the feet to allow the test subject to enter and exit the system. While the rings are in this position and e.g. not obstructing the modules 400, the model/point cloud may be obtained and visible and thermal imaging can be performed. In another embodiment, the location of the vibrometers 1500 1502 may be as illustrated in figure 5.

After the model/point cloud acquisition, full body colour image and/or thermal images acquisition may be performed, after which the rings 104 and 106 may move using rails 1600 over the test subjects body to automatically position the first and second vibration sensors.

In an embodiment, a ring can be positioned over the thorax and a vibration sensor comprising a 2D array of vibrometers can be used to acquire heart movements and heart sounds. The rings 104 and 106 may then move into position over the neck and pelvis, respectively to measure the PWV. For example, laser vibrometers 1500 in ring 104 may measure the carotid pulse wave and the microwave radar vibrometers 1502 in ring 106 may measure the femoral pulse.

In an embodiment, the vibrometer array 1500 of the ring 104 located over the neck area may also detect the amplitude of the movement of the wall of the common carotid artery as a result of the pulse wave. Such a movement may be collected and analysed over multiple heart beats, if desired. If the movement is large, then the artery walls are pliable and the PWV should be lower. This direct measurement of the stiffness of the arteries may be combined with the cfPWV measurements and/or the direct 2D array 804 measurements of the PWV.

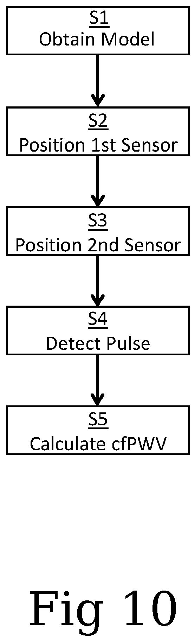

Figure 10 is a flow chart illustrating some embodiments of the method of the present disclosure. The method is for calculating a cfPWV in a live body B of a human or animal.

The method comprises obtaining S1 a model of the body, e.g. the model consisting of or comprising a point cloud, of the body. Thus, in some embodiments, a point cloud is acquired, e.g. as discussed above.

The method further comprises automatically positioning S2 a first vibration sensor, e.g. 1302 or 1500, over a neck area N of the body B, based on the obtained S1 model. By means of the model, the first vibration sensor can be automatically positioned without the need for involvement of a human operator. In some embodiments, e.g. when thermal imaging was part of the scanning, the first vibration sensor may be automatically positioned over the common carotid artery 1400 within the neck area N based on the thermal imaging. Preferably, the first vibration sensor comprises at least one laser vibrometer, laser vibrometers having high sensitivity. For instance, the first vibration sensor may comprise a plurality of vibrometers, e.g. laser vibrometers, e.g. arranged as a 1D or 2D an array of vibrometers.

The method further comprises automatically positioning S3 a second vibration sensor, e.g. 1304 or 1502, over a pelvis area P of the body B, based on the obtained S1 model. By means of the model, the second vibration sensor can be automatically positioned without the need for involvement of a human operator. In some embodiments, e.g. when thermal imaging was part of the obtaining S1 of the model, the second vibration sensor may be automatically positioned over one of the femoral arteries 1406 within the pelvis area P based on the thermal imaging. Preferably, the second vibration sensor comprises at least one laser vibrometer, laser vibrometers having high sensitivity, or at least one microwave radar vibrometer, microwave radar having the advantage of being usable even if the subject has its underwear on. For instance, the second vibration sensor may comprise a plurality of vibrometers, e.g. laser and/or microwave vibrometers, e.g. arranged as a 1D or 2D an array of vibrometers.

Then the method comprises detecting S4 a carotid artery 1400 pulse by means of the first vibration sensor and a femoral artery 1406 pulse by means of the second vibration sensor, both the detected carotid artery pulse and the detected femoral artery pulse resulting from the same blood pressure pulse wave. That they result from the same pulse wave implies that they are created by the same hart contraction, allowing the cfPWV to be calculated based on the detection of the artery pulses.

In some embodiments, a single direction controllable vibrometer 1302, 1304, e.g. laser or microwave vibrometer, is used in either or both of the first and second vibration sensors. The direction and thus the location of the spot pickup area on the neck or pelvis may therefore be adjusted to be as close as possible to the artery. This adjustment can be done using the obtained model. In some embodiments, the adjustment is done using thermal images previously taken by a thermal cameras 310. The blood in the artery may give a measurable heat signature in the thermal image and can therefore be used to guide the vibrometer pickup spot to the best location.

In other embodiments, 1D arrays 1500, 1502 of vibrometers are used. The advantage of using a 1D array is that the exact pickup spot location does not need to be known as precisely, it may be enough to position the array over the line made by a transvers plane through the neck area N. The strongest carotid pulse detected by one of the vibrometers within the 1D array of vibrometers may then conveniently be used.

In other embodiments, 2D arrays 804 of vibrometers may be used. With such an array it may be possible to follow the pulse moving from sensor 808 to 810 and thus it may be possible to measure the pulse wave velocity directly 2304 from the carotid artery and/or similarly from the femoral artery. When performing a direct local measurement like this, the carotid artery may be of more significance than the femoral artery, since the latter may be more affected by bad sleep, coffee and muscular tension. It may be possible to measure the PWV directly in the carotid artery and/or the femoral artery by means of a 1D array of at least two vibrometers, but it may be difficult to align a 1D array sufficiently with the artery to allow this, why a 2D array of vibrometers may be preferred. In some embodiments, a 2D array 802 may be used to track both a transvers and longitudinal movement of the pulse wave. This may be necessary since the artery is usually not perfectly aligned with the longitudinal direction of the 2D array.

Thus, the method of the present disclosure may in some embodiments comprise detecting the carotid artery pulse by at least two, spatially distanced, vibrometers of the plurality of vibrometers of the first vibration sensor, and calculating a local carotid PWV by means of said at least two vibrometers of the first vibration sensor. Additionally or alternatively, the method of the present disclosure may in some embodiments comprise detecting the femoral artery pulse by at least two, spatially distanced, vibrometers of the plurality of vibrometers of the second vibration sensor, and calculating a local femoral PWV by means of said at least two vibrometers of the second vibration sensor. The local carotid PWV and the local femoral PWV may be used in combination with the calculated cfPWV to obtain an isolated PWV of the aorta. In some cases, the aortic PWV is sought, e.g. to determine stiffness thereof, but this may be difficult to measure directly, why the cfPWV is used instead. By removing the contributions to the cfPWV of the local carotid and femoral PWV, the PWV of the aorta may be isolated. Thus, in some embodiments, the method of the present disclosure may comprise estimating an aortic PWV based on the calculated cfPWV in view of the calculated local carotid PWV and the calculated local femoral PWV. It may further improve the estimation of the aortic PWV if the respective spatial distance between where the carotid artery pulse is detected and where the carotid artery joins the aorta, and between where the femoral artery pulse is detected and where the femoral artery joins the aorta. These spatial distances may be determined or estimated from the obtained geometrical model of the body.

Then the method comprises calculating S5 the carotid-femoral PWV (cfPWV), based on a time difference between the detected carotid pulse and the detected femoral pulse, and on a spatial distance between the neck area and the pelvis area, e.g. the distance between where the carotid artery pulse was detected and where the femoral artery pulse was detected, if those places are known. As mentioned above, 80% of this distance may be used in the calculation S5, or a more precise or individualized calculation of the distance may be performed, to obtain the distance travelled from the heart by the pulse wave.

If a plurality of vibrometers are used in the first and/or second vibration sensor, e.g. forming a 1D or 2D array 1500, 1502; 802 of vibrometers, a vibrometer of said plurality which detects the strongest carotid pulse response may be used for the detecting of the carotid or femoral artery pulse.

As mentioned above, when using a plurality of vibrometers in the first and/or second vibration sensor, the local PWV in the carotid and/or femoral artery may be detected, especially when a 2D array of vibrometers are used. Thus, in some embodiments, the method may further comprise detecting the carotid or femoral artery pulse by at least two, spatially distanced, vibrometers of the plurality of vibrometers of the first or second vibration sensor, and calculating the local carotid or femoral PWV by means of said at least two vibrometers of the first or second vibration sensor.

Since the body B has two common carotid arteries, one on each side of the neck N, and two femoral arteries, one on each side of the pelvis P, it may be convenient to detect artery pulses in both to improve correctness and redundancy of the calculation of the cfPWV. Thus, in some embodiments of the present invention, the method comprises detecting the carotid artery pulse in both carotid arteries, and/or detecting the femoral artery pulse in both femoral arteries.

When detecting the pulse in the carotid artery, there may be interference from the jugular vein which lies close to the carotid in the neck and which also has a pulse albeit weaker and less defined, especially when the body is horizontal. If the back of the subject's body is raised such that the neck becomes elevated in relation to the hart, the interference from the jugular is reduced. Thus, in some embodiments of the present invention, the upper body of the live body may be raised, e.g. by bending at the hip, during the detecting of the carotid artery pulse (and thus also of the femoral artery pulse), e.g. to an angle (e.g. back angle) within the range of 20-60 degrees to the horizontal plane, such as within the range of 30-45 degrees. This implies that the body, or at least the upper body thereof, is positioned at said angle to the horizontal plane during detection of the pulses. If the angle of the upper body is obtained by the body bending at the hip, detection of the femoral artery pulse may be impaired if the angle is too high, e.g. above 60 or 45 degrees. An alternative is to detect the pulses when the body is upright, e.g. when the subject is standing rather than reclining.

For efficiency and to reduce the need for sterilization of equipment before or after use, it is advantageous to not allow the first and/or second vibrations sensors, or any frame to which it is mounted, to come into physical contact with the body. The subject may e.g. simply walk up to equipment mounted on a frame and stand still while the sensor data is acquired from the body of the subject and while the pulses are detected, and then walk away to allow a next subject to walk up to the equipment. Similarly, if the equipement is mounted above a bed for a reclining subject, the equipment is preferably mounted high enough above the bed to allow the subject to lie down on and get up from the bed without impacting the equipement. During detection of the artery and femoral pulses, the first and/or second, preferably both, vibration sensor may thus be positioned at a distance within the range of 10-100 cm, e.g. 30-50 cm, from the body, implying that e.g. the laser of laser vibrometers or microwaves of microwave vibrometers in either of the vibration sensors travel through air said distance before encountering the skin of the body. Thus, in some embodiments of the present invention, the detecting of the carotid artery pulse and the femoral artery pulse is done contactlessly, without physical contact between the first vibration sensor and the body and/or between the second vibration sensor and the body, e.g. at a distance within the range of 10-100 cm, e.g. 30-50 cm, between the first or second vibration sensor and the body.

The present disclosure has mainly been described above with reference to a few embodiments. However, as is readily appreciated by a person skilled in the art, other embodiments than the ones disclosed above are equally possible within the scope of the present disclosure, as defined by the appended claims.

## Claims

1. A method of calculating a Pulse Wave Velocity, PWV, in a live body (B), the method comprising:
obtaining (S1) a geometrical model of the body based on acquired sensor data;
based on the obtained (S1) model, automatically positioning (S2) a first vibration sensor (1302; 1500) in relation to a neck area (N) of the body;
based on the obtained (S1) model, automatically positioning (S3) a second vibration sensor (1304; 1502) in relation to a pelvis area (P) of the body;
detecting (S4) a carotid artery (1400) pulse by means of the first vibration sensor and a femoral artery (1406) pulse by means of the second vibration sensor, both the detected carotid artery pulse and the detected femoral artery pulse resulting from the same blood pressure pulse wave; and
calculating (S5) the carotid-femoral PWV, cfPWV, based on a time difference between the detected carotid pulse and the detected femoral pulse, and on a spatial distance between the neck area and the pelvis area.

2. The method of claim 1,
wherein the first vibration sensor comprises a laser vibrometer, e.g. wherein the first vibration sensor is a 1D array (1500) of laser vibrometers or a 2D array (802) of laser vibrometers, and/or
wherein the second vibration sensor comprises a microwave radar vibrometer, e.g. wherein the second vibration sensor is a 1D array (1502) of microwave radars or a 2D array (802) of microwave radars.

3. The method of any preceding claim, wherein the first vibration sensor comprises a plurality of vibrometers, e.g. laser vibrometers, such as a 1D or 2D array of vibrometers.

4. The method of claim 3, wherein only the vibrometer, of said plurality of vibrometers, which detects the strongest carotid pulse response is used for the detecting of the carotid artery pulse.

5. The method of claim 3 or 4, further comprising:
detecting the carotid artery pulse by at least two, spatially distanced, vibrometers of the plurality of vibrometers of the first vibration sensor; and
calculating a local carotid PWV by means of said at least two vibrometers of the first vibration sensor.

6. The method of any preceding claim, wherein the second vibration sensor comprises a second plurality of vibrometers, e.g. microwave radars, such as a 1D or 2D array of vibrometers.

7. The method of claim 6, wherein only the vibrometer, of said second plurality of vibrometers, which detects the strongest femoral pulse response is used for the detecting of the femoral artery pulse.

8. The method of claim 6 or 7, further comprising:
detecting the femoral artery pulse by at least two, spatially distanced, vibrometers of the plurality of vibrometers of the second vibration sensor; and
calculating a local femoral PWV by means of said at least two vibrometers of the second vibration sensor.

9. The method of claim 1, wherein
the first vibration sensor comprises a plurality of vibrometers, e.g. laser vibrometers, such as a 1D or 2D array of vibrometers, the method further comprising:
detecting the carotid artery pulse by at least two, spatially distanced, vibrometers of the plurality of vibrometers of the first vibration sensor, and
calculating a local carotid PWV by means of said at least two vibrometers of the first vibration sensor; and
the second vibration sensor comprises a second plurality of vibrometers, e.g. microwave radars, such as a 1D or 2D array of vibrometers, the method further comprising:
detecting the femoral artery pulse by at least two, spatially distanced, vibrometers of the plurality of vibrometers of the second vibration sensor, and
calculating a local femoral PWV by means of said at least two vibrometers of the second vibration sensor;
further comprising:
estimating an aortic PWV based on the calculated (S5) cfPWV in view of the calculated local carotid PWV and the calculated local femoral PWV.

10. The method of claim 1, wherein the first vibration sensor and/or the second vibration sensor, preferably the second vibration sensor, comprises a 1D or 2D array of microwave radar vibrometers, each operating at a frequency within the range of 80 200 GHz, preferably within the range of 120-150 GHz, e.g. within the range of 136 148 GHz.

11. The method of claim 10, wherein each of the microwave radar vibrometers is provided with a microwave lens for narrowing a microwave beam emitted by the vibrometer towards the area of the body in relation to which the vibration sensor is positioned (S2/S3).

12. The method of claim 10 or 11, wherein the positioning (S2/S3) comprises positioning the vibration sensor at a distance from the area of the body in relation to which the vibration sensor is positioned such that for each of the microwave radar vibrometers, its pick-up area on the area of the body overlaps with the respective pick-up area(s) of its adjacent microwave radar vibrometer(s) in the array.

13. The method of any preceding claim,
wherein the detecting of the carotid artery pulse comprises detecting the pulse in both carotid arteries, on respective sides of the neck, and/or
wherein the detecting of the femoral artery pulse comprises detecting the pulse in both femoral arteries, on respective sides of the pelvis.

14. The method of any preceding claim, wherein the upper body of the live body is raised, e.g. by bending at the hip, during the detecting of the carotid artery pulse and the femoral artery pulse, e.g. to an angle within the range of 20-60 degrees to the horizontal plane, such as within the range of 30-45 degrees.

15. The method of any preceding claim,
wherein the positioning of the first vibration sensor comprises guiding the first vibration sensor by means of an obtained thermal image of the neck area, and/or
wherein the positioning of the second vibration sensor comprises guiding the second vibration sensor by means of an obtained thermal image of the pelvis area.

16. The method of any preceding claim, wherein the detecting of the carotid artery pulse and the femoral artery pulse is done contactlessly, without physical contact between the first vibration sensor and the body and/or between the second vibration sensor and the body.

17. The method of claim 16, wherein the positioning (S2/S3) of the first vibration sensor and/or the second vibration sensor comprises positioning the vibration sensor at a distance from the area of the body in relation to which the vibration sensor is positioned which is within the range of 10-100 cm, e.g. 30-50 cm.

18. The method of any preceding claim, wherein the model is or comprises a point cloud of the body.

19. The method of any preceding claim, wherein the first vibration sensor comprises at least one laser vibrometer, and wherein the positioning (S2) of the first vibration sensor comprises positioning a pick-up spot of the laser vibrometer on the skin of the body by means of a movable mirror.

20. A system (100) comprising:
means configured for obtaining (S1) a geometrical model of a live body (B);
means configured for, based on the obtained (S1) model, automatically positioning (S2) a first vibration sensor (1302; 1500) in relation to a neck area (N) of the body;
means configured for, based on the obtained (S1) model, automatically positioning (S3) a second vibration sensor (1304; 1502) in relation to a pelvis area (P) of the body;
means configured for detecting (S4) a carotid artery (1400) pulse by means of the first vibration sensor and a femoral artery (1406) pulse by means of the second vibration sensor, both the detected carotid artery pulse and the detected femoral artery pulse resulting from the same blood pressure pulse wave; and
means configured for calculating (S5) the the carotid-femoral pulse wave velocity, cfPWV, based on a time difference between the detected carotid pulse and the detected femoral pulse, and on a spatial distance between the neck area and the pelvis area.

## Patentansprüche

1. Verfahren zum Berechnen einer Pulswellengeschwindigkeit, PWV, in einem lebenden Körper (B), wobei das Verfahren Folgendes umfasst:
Gewinnen (S1) eines geometrischen Modells des Körpers auf Grundlage von erfassten Sensordaten,
auf Grundlage des gewonnenen (S1) Modells automatisches Positionieren (S2) eines ersten Vibrationssensors (1302; 1500) im Verhältnis zu einem Halsbereich (N) des Körpers,
auf Grundlage des gewonnenen (S1) Modells automatisches Positionieren (S3) eines zweiten Vibrationssensors (1304; 1502) im Verhältnis zu einem Beckenbereich (P) des Körpers,
Erfassen (S4) eines Karotisarterien- (1400) Pulses mit Hilfe des ersten Vibrationssensors und eines Femoralarterien- (1406) Pulses mit Hilfe des zweiten Vibrationssensors, wobei sich sowohl der erfasste Karotisarterien-Puls als auch der erfasste Femoralarterien-Puls aus derselben Blutdruck-Pulswelle ergeben, und
Berechnen (S5) der Carotis-Femoralis-Pulswellengeschwindigkeit, cfPWV, auf Grundlage einer zeitlichen Differenz zwischen dem erfassten Carotis-Puls und dem erfassten Femoralis-Puls und eines räumlichen Abstandes zwischen dem Halsbereich und dem Beckenbereich.

2. Verfahren nach Anspruch 1,
wobei der erste Vibrationssensor ein Laservibrometer umfasst, z. B., wobei der erste Vibrationssensor eine 1D-Gruppierung (1500) von Laservibrometern oder eine 2D-Gruppierung (802) von Laservibrometern ist und/oder
wobei der zweite Vibrationssensor ein Mikrowellenradar-Vibrometer umfasst, z. B., wobei der erste Vibrationssensor eine 1D-Gruppierung (1502) von Mikrowellenradars oder eine 2D-Gruppierung (802) von Mikrowellenradars ist.

3. Verfahren nach einem der vorhergehenden Ansprüche, wobei der erste Vibrationssensor eine Vielzahl von Vibrometern, z. B. Laservibrometern, wie beispielsweise eine 1D- oder 2D-Gruppierung von Vibrometern, umfasst.

4. Verfahren nach Anspruch 3, wobei nur dasjenige Vibrometer, von der Vielzahl von Vibrometern, das die stärkste Carotis-Puls-Antwort erfasst, für das Erfassen des Karotisarterien-Pulses verwendet wird.

5. Verfahren nach Anspruch 3 oder 4, das ferner Folgendes umfasst:
Erfassen des Karotisarterien-Pulses durch mindestens zwei, räumlich beabstandete, Vibrometer von der Vielzahl von Vibrometern des ersten Vibrationssensors und
Berechnen einer örtlichen Carotis-PWV mit Hilfe der mindestens zwei Vibrometer des ersten Vibrationssensors.

6. Verfahren nach einem der vorhergehenden Ansprüche, wobei der zweite Vibrationssensor eine zweite Vielzahl von Vibrometern, z. B. Mikrowellenradars, wie beispielsweise eine 1D- oder 2D-Gruppierung von Vibrometern, umfasst.

7. Verfahren nach Anspruch 6, wobei nur dasjenige Vibrometer, von der zweiten Vielzahl von Vibrometern, das die stärkste Femoralis-Puls-Antwort erfasst, für das Erfassen des Femoralarterien-Pulses verwendet wird.

8. Verfahren nach Anspruch 6 oder 7, das ferner Folgendes umfasst:
Erfassen des Femoralarterien-Pulses durch mindestens zwei, räumlich beabstandete, Vibrometer von der Vielzahl von Vibrometern des zweiten Vibrationssensors und
Berechnen einer örtlichen Femoralis-PWV mit Hilfe der mindestens zwei Vibrometer des zweiten Vibrationssensors.

9. Verfahren nach Anspruch 1, wobei
der erste Vibrationssensor eine Vielzahl von Vibrometern, z. B. Laservibrometern, wie beispielsweise eine 1D- oder 2D-Gruppierung von Vibrometern, umfasst, wobei das Verfahren ferner Folgendes umfasst:
Erfassen des Karotisarterien-Pulses durch mindestens zwei, räumlich beabstandete, Vibrometer von der Vielzahl von Vibrometern des ersten Vibrationssensors und
Berechnen einer örtlichen Carotis-PWV mit Hilfe der mindestens zwei Vibrometer des ersten Vibrationssensors und
wobei der zweite Vibrationssensor eine zweite Vielzahl von Vibrometern, z. B. Mikrowellenradars, wie beispielsweise eine 1D- oder 2D-Gruppierung von Vibrometern, umfasst, wobei das Verfahren ferner Folgendes umfasst:
Erfassen des Femoralarterien-Pulses durch mindestens zwei, räumlich beabstandete, Vibrometer von der Vielzahl von Vibrometern des zweiten Vibrationssensors und
Berechnen einer örtlichen Femoralis-PWV mit Hilfe der mindestens zwei Vibrometer des zweiten Vibrationssensors,
ferner umfassend:
Schätzen einer Aorten-PWV auf Grundlage der berechneten (S5) cfPWV im Hinblick auf die berechnete örtliche Carotis-PWV und die berechnete örtliche Femoralis-PWV.

10. Verfahren nach Anspruch 1, wobei der erste Vibrationssensor und/oder der zweite Vibrationssensor, vorzugsweise der zweite Vibrationssensor, eine 1D- oder 2D-Gruppierung von Mikrowellenradar-Vibrometern umfasst, die jeweils bei einer Frequenz innerhalb des Bereichs von 80 bis 200 GHz, vorzugsweise innerhalb des Bereichs von 120 bis 150 GHz, z. B. innerhalb des Bereichs von 136 bis 148 GHz, arbeiten.

11. Verfahren nach Anspruch 10, wobei jedes von den Mikrowellenradar-Vibrometern mit einer Mikrowellenlinse zum Verengen eines Mikrowellenstrahls, der durch das Vibrometer emittiert wird, hin zu dem Bereich des Körpers, im Verhältnis zu dem der Vibrationssensor positioniert (S2/S3) ist, versehen ist.

12. Verfahren nach Anspruch 10 oder 11, wobei das Positionieren (S2/S3) das Positionieren des Vibrationssensors bei einem Abstand von dem Bereich des Körpers, im Verhältnis zu dem der Vibrationssensor positioniert wird, derart umfasst, dass für jedes von den Mikrowellenradar-Vibrometern sich sein Aufnahmebereich auf dem Bereich des Körpers mit dem/den jeweiligen Aufnahmebereich(en) seines/seiner benachbarten Mikrowellenradar-Vibrometer in der Gruppierung überlappt.

13. Verfahren nach einem der vorhergehenden Ansprüche,
wobei das Erfassen des Karotisarterien-Pulses das Erfassen des Pulses in beiden Karotisarterien, auf jeweiligen Seiten des Halses, umfasst und/oder
wobei das Erfassen des Femoralarterien-Pulses das Erfassen des Pulses in beiden Femoralarterien, auf jeweiligen Seiten des Beckens, umfasst.

14. Verfahren nach einem der vorhergehenden Ansprüche, wobei der Oberkörper des lebenden Körpers während des Erfassens des Karotisarterien-Pulses und des Femoralarterien-Pulses angehoben wird, z. B. durch Beugen an der Hüfte, z. B. bis zu einem Winkel innerhalb des Bereichs von 20 bis 60 Grad zu der horizontalen Ebene, wie beispielsweise innerhalb des Bereichs von 30 bis 45 Grad.

15. Verfahren nach einem der vorhergehenden Ansprüche,
wobei das Positionieren des ersten Vibrationssensors das Führen des ersten Vibrationssensors mit Hilfe eines gewonnenen Wärmebildes des Halsbereichs umfasst und/oder
wobei das Positionieren des zweiten Vibrationssensors das Führen des zweiten Vibrationssensors mit Hilfe eines gewonnenen Wärmebildes des Beckenbereichs umfasst.

16. Verfahren nach einem der vorhergehenden Ansprüche, wobei das Erfassen des Karotisarterien-Pulses und des Femoralarterien-Pulses berührungsfrei, ohne physische Berührung zwischen dem ersten Vibrationssensor und dem Körper und/oder zwischen dem zweiten Vibrationssensor und dem Körper, vorgenommen wird.

17. Verfahren nach Anspruch 16, wobei das Positionieren (S2/S3) des ersten Vibrationssensors und/oder des zweiten Vibrationssensors das Positionieren des Vibrationssensors bei einem Abstand von dem Bereich des Körpers, im Verhältnis zu dem der Vibrationssensor positioniert wird, umfasst, der innerhalb des Bereichs von 10 bis 100 cm, z. B. 30 bis 50 cm, liegt.

18. Verfahren nach einem der vorhergehenden Ansprüche, wobei das Modell eine Punktwolke des Körpers ist oder umfasst.

19. Verfahren nach einem der vorhergehenden Ansprüche, wobei der erste Vibrationssensor mindestens ein Laservibrometer umfasst und wobei das Positionieren (S2) des ersten Vibrationssensors das Positionieren eines Aufnahmeortes des Laservibrometers auf der Haut des Körpers mit Hilfe eines beweglichen Spiegels umfasst.

20. System (100), das Folgendes umfasst:
Mittel, die zum Gewinnen (S1) eines geometrischen Modells eines lebenden Körpers (B) konfiguriert sind,
Mittel, die, auf Grundlage des gewonnenen (S1) Modells, zum automatischen Positionieren (S2) eines ersten Vibrationssensors (1302; 1500) im Verhältnis zu einem Halsbereich (N) des Körpers konfiguriert sind,
Mittel, die, auf Grundlage des gewonnenen (S1) Modells, zum automatischen Positionieren (S3) eines zweiten Vibrationssensors (1304; 1502) im Verhältnis zu einem Beckenbereich (P) des Körpers konfiguriert sind,
Mittel, die zum Erfassen (S4) eines Karotisarterien-(1400) Pulses mit Hilfe des ersten Vibrationssensors und eines Femoralarterien- (1406) Pulses mit Hilfe des zweiten Vibrationssensors konfiguriert sind, wobei sich sowohl der erfasste Karotisarterien-Puls als auch der erfasste Femoralarterien-Puls aus derselben Blutdruck-Pulswelle ergeben, und
Mittel, die zum Berechnen (S5) der Carotis-Femoralis-Pulswellengeschwindigkeit, cfPWV, auf Grundlage einer zeitlichen Differenz zwischen dem erfassten Carotis-Puls und dem erfassten Femoralis-Puls und eines räumlichen Abstandes zwischen dem Halsbereich und dem Beckenbereich konfiguriert sind.

## Revendications

1. Procédé de calcul d'une vitesse d'onde de pouls, PWV - Pulse Wave Velocity, dans un corps vivant (B), le procédé comprenant :
l'obtention (S1) d'un modèle géométrique du corps basé sur des données acquises par capteur ;
sur la base du modèle obtenu (S1), le positionnement (S2) automatique d'un premier capteur de vibrations (1302 ; 1500) par rapport à une zone du cou (N) du corps ;
sur la base du modèle obtenu (S1), le positionnement (S3) automatique d'un second capteur de vibrations (1304 ; 1502) par rapport à une zone du bassin (P) du corps ;
la détection (S4) d'un pouls d'artère carotide (1400) au moyen du premier capteur de vibrations et d'un pouls d'artère fémorale (1406) au moyen du second capteur de vibrations, le pouls d'artère carotide détecté et le pouls d'artère fémorale détecté résultant tous deux de la même onde de pouls de tension artérielle ; et
le calcul (S5) de la vitesse d'onde de pouls carotidien-fémoral, cfPWV, sur la base d'une différence de temps entre le pouls carotidien détecté et le pouls fémoral détecté, et d'une distance dans l'espace entre la zone du cou et la zone du bassin.

2. Procédé selon la revendication 1,
dans lequel le premier capteur de vibrations comprend un vibromètre laser, par exemple dans lequel le premier capteur de vibrations est un réseau 1D (1500) de vibromètres laser ou un réseau 2D (802) de vibromètres laser, et/ou
dans lequel le second capteur de vibrations comprend un vibromètre radar à micro-ondes, par exemple dans lequel le second capteur de vibrations est un réseau 1D (1502) de radars à micro-ondes ou un réseau 2D (802) de radars à micro-ondes.

3. Procédé selon l'une des revendications précédentes, dans lequel le premier capteur de vibrations comprend une pluralité de vibromètres, par exemple des vibromètres laser, tels qu'un réseau 1D ou 2D de vibromètres.

4. Procédé selon la revendication 3, dans lequel seul le vibromètre de ladite pluralité de vibromètres qui détecte la réponse de pouls carotidien la plus forte est utilisé pour détecter le pouls d'artère carotide.

5. Procédé selon la revendication 3 ou 4, comprenant en outre :
la détection du pouls d'artère carotide par au moins deux vibromètres, distancés dans l'espace, de la pluralité de vibromètres du premier capteur de vibrations ; et
le calcul d'une vitesse d'onde de pouls carotidien local au moyen desdits au moins deux vibromètres du premier capteur de vibrations.

6. Procédé selon l'une des revendications précédentes, dans lequel le second capteur de vibrations comprend une seconde pluralité de vibromètres, par exemple des radars à micro-ondes, tels qu'un réseau 1D ou 2D de vibromètres.

7. Procédé selon la revendication 6, dans lequel seul le vibromètre de ladite seconde pluralité de vibromètres qui détecte la réponse de pouls fémoral la plus forte est utilisé pour la détection du pouls d'artère fémorale.

8. Procédé selon la revendication 6 ou 7, comprenant en outre :
la détection du pouls d'artère fémorale par au moins deux vibromètres, distancés dans l'espace, de la pluralité de vibromètres du second capteur de vibrations ; et
le calcul d'une vitesse d'onde de pouls fémoral local au moyen desdits au moins deux vibromètres du second capteur de vibrations.

9. Procédé selon la revendication 1, dans lequel
le premier capteur de vibrations comprend une pluralité de vibromètres, par exemple des vibromètres laser, tels qu'un réseau 1D ou 2D de vibromètres, le procédé comprenant en outre :
la détection du pouls d'artère carotide par au moins deux vibromètres, distancés dans l'espace, de la pluralité de vibromètres du premier capteur de vibrations, et
le calcul d'une vitesse d'onde de pouls carotidien local au moyen desdits au moins deux vibromètres du premier capteur de vibrations ; et
le second capteur de vibrations comprend une seconde pluralité de vibromètres, par exemple des radars à micro-ondes, tels qu'un réseau 1D ou 2D de vibromètres, le procédé comprenant en outre :
la détection du pouls d'artère fémorale par au moins deux vibromètres, distancés dans l'espace, de la pluralité de vibromètres du second capteur de vibrations, et
le calcul d'une vitesse d'onde de pouls fémoral local au moyen desdits au moins deux vibromètres du second capteur de vibrations ;
comprenant en outre :
l'estimation d'une vitesse d'onde de pouls aortique sur la base de la cfPWV calculée (S5) compte tenu de la vitesse d'onde de pouls carotidien local calculée et de la vitesse d'onde de pouls fémoral local calculée.

10. Procédé selon la revendication 1, dans lequel le premier capteur de vibrations et/ou le second capteur de vibrations, de préférence le second capteur de vibrations, comprend un réseau 1D ou 2D de vibromètres radar à micro-ondes, chacun fonctionnant à une fréquence comprise dans une plage de 80 200 GHz, de préférence dans une plage de 120-150 GHz, par exemple dans une plage de 136 148 GHz.

11. Procédé selon la revendication 10, dans lequel chacun des vibromètres radar à micro-ondes est fourni avec une lentille micro-ondes pour limiter un rayon de micro-ondes émis par le vibromètre vers la zone du corps par rapport à laquelle le capteur de vibrations est positionné (S2/S3).

12. Procédé selon la revendication 10 ou 11, dans lequel le positionnement (S2/S3) comprend le positionnement du capteur de vibrations à une distance de la zone du corps par rapport à laquelle le capteur de vibrations est positionné, de manière à ce que pour chacun des vibromètres radar à micro-ondes, sa zone de balayage sur la zone du corps chevauche la (les) zone(s) de balayage de son (ses) vibromètre(s) radar à micro-ondes adjacent(s) dans le réseau.

13. Procédé selon l'une des revendications précédentes,
dans lequel la détection du pouls de l'artère carotide comprend la détection du pouls dans les deux artères carotides, sur des côtés respectifs du cou, et/ou
dans lequel la détection du pouls de l'artère fémorale comprend la détection du pouls dans les deux artères fémorales, sur des côtés respectifs du bassin.

14. Procédé selon l'une des revendications précédentes, dans lequel le haut du corps du corps vivant est levé, par exemple par flexion au niveau de la hanche, pendant la détection du pouls d'artère carotide et du pouls d'artère fémorale, par exemple à un angle situé dans une plage de 20-60 degrés par rapport au plan horizontal, tel que dans une plage de 30-45 degrés.

15. Procédé selon l'une des revendications précédentes,
dans lequel le positionnement du premier capteur de vibrations comprend un guidage du premier capteur de vibrations au moyen d'une image thermique obtenue de la zone du cou, et/ou dans lequel le positionnement du second capteur de vibrations comprend un guidage du second capteur de vibrations au moyen d'une image thermique obtenue de la zone du bassin.

16. Procédé selon l'une des revendications précédentes, dans lequel la détection du pouls d'artère carotide et du pouls d'artère fémorale est effectué sans contact, sans contact physique entre le premier capteur de vibrations et le corps et/ou entre le second capteur de vibrations et le corps.

17. Procédé selon la revendication 16, dans lequel le positionnement (S2/S3) du premier capteur de vibrations et/ou du second capteur de vibrations comprend un positionnement du capteur de vibrations à distance de la zone du corps par rapport à laquelle le capteur de vibrations est positionné, distance qui se situe dans une plage de 10-100 cm, par exemple 30-50 cm.

18. Procédé selon l'une des revendications précédentes, dans lequel le modèle est ou comprend un nuage de points du corps.

19. Procédé selon l'une des revendications précédentes, dans lequel le premier capteur de vibrations comprend au moins un vibromètre laser, et dans lequel le positionnement (S2) du premier capteur de vibrations comprend un positionnement d'un point de balayage du vibromètre laser sur la peau du corps au moyen d'un miroir mobile.

20. Système (100) comprenant :
des moyens configurés pour obtenir (S1) un modèle géométrique d'un corps vivant (B) ;
des moyens configurés pour positionner (S2) automatiquement, sur la base du modèle obtenu (S1), un premier capteur de vibrations (1302 ; 1500) par rapport à une zone du cou (N) du corps ;
des moyens configurés pour positionner (S3) automatiquement, sur la base du modèle obtenu (S1), un second capteur de vibrations (1304 ; 1502) par rapport à une zone du bassin (P) du corps ;
des moyens configurés pour détecter (S4) un pouls d'artère carotide (1400) au moyen du premier capteur de vibrations et un pouls d'artère fémorale (1406) au moyen du second capteur de vibrations, le pouls d'artère carotide détecté et le pouls d'artère fémorale détecté résultant tous deux de la même onde de pouls de tension artérielle ; et
des moyens configurés pour calculer (S5) la vitesse d'onde de pouls carotidien-fémoral, cfPWV, sur la base d'une différence de temps entre le pouls carotidien détecté et le pouls fémoral détecté, et d'une distance dans l'espace entre la zone du cou et la zone du bassin.
